# FASCICULE DE BREVET EUROPEEN

(11) **EP 2 324 801 B1**
(45) Date de publication et mention de la délivrance du brevet: **12.05.2021**
(21) Numéro de dépôt: 10191949.6
(22) Date de dépôt: 19.11.2010
(51) Int. Cl.: A61F 2/46, A61F 2/40, A61B 5/107, G06T 17/00, A61B 5/103, A61B 17/15, G06T 7/60, A61B 5/00, A61F 2/30, A61F 2/48, A61B 34/10, A61B 90/00, A61B 34/20

(54) **Dispositif et procédé de détermination d'une configuration d'implantation d'un composant prothétique glénoïdien ou d'une configuration d'application d'un outil de resurfaçage glénoïdien**
Vorrichtung und Verfahren zur Bestimmung einer Implantationskonfiguration eines Glenoidprothesenteils oder einer Anwendungskonfiguration eines Werkzeugs zur Wiederherstellung der Glenoidoberfläche
Device and method for determining a configuration for implanting a glenoid prosthetic component or a configuration for applying a glenoid resurfacing tool

(30) Priorité: 27.01.2010 FR 1050541; 24.11.2009 US 264027 P
(43) Date de publication de la demande: 25.05.2011
(73) Titulaire: Tornier, 38330 Montbonnot-Saint-Martin (FR)
(72) Inventeur: Ratron, Yves-Alain, 38000, Grenoble (FR)
(74) Mandataire: Lavoix

(56) Documents cités:
- EP-A1- 2 135 576
- EP-A2- 1 249 213
- US-A- 5 610 966
- US-A1- 2005 197 814
- US-A1- 2007 089 518
- US-A1- 2008 243 127
- US-A1- 2009 226 068

## Description

La présente invention concerne un dispositif et un procédé de détermination d'une configuration d'implantation chez un patient d'un composant glénoïdien d'une prothèse d'épaule. Elle concerne également un dispositif et un procédé de détermination d'une configuration d'application d'un outil de resurfaçage glénoïdien.

Le remplacement de la surface articulaire glénoïdienne de l'omoplate d'un être humain par un composant glénoïdien d'une prothèse d'épaule est une opération chirurgicale délicate, notamment en raison de l'environnement musculaire de l'épaule. On constate que, selon la position d'implantation d'un tel composant glénoïdien, des risques de descellement du composant existent en raison des efforts appliqués à ce composant lors des mouvements ultérieurs de l'épaule prothésée.

En particulier, chez certains patients, on a constaté que, même si l'implantation sur leur omoplate d'un tel composant glénoïdien était parfaitement centrée sur la tête articulaire de l'humérus correspondant à l'issue de l'intervention chirurgicale d'implantation, la reprise de leurs activités conduisait, plus ou moins rapidement, à une instabilité de la prothèse. Une explication tiendrait au fait que l'implantation du composant glénoïdien n'a pas été réalisée en tenant compte de forces d'origine musculaire générées régulièrement par le patient dans le cadre de ses activités de tous les jours.

Pour contourner cette problématique, US-A-2009/0226068 propose de fabriquer un composant glénoïdien « sur mesure » dont la surface articulaire est conformée, lors de sa conception, en tenant compte de l'usure constatée sur la glène à prothéser d'un patient. Une telle approche s'avère cependant onéreuse puisqu'elle conduit à personnaliser le composant glénoïdien pour chaque patient à traiter. De plus, US-A-2009/0226068 prévoit d'implanter ces composants glénoïdiens « sur mesure » de manière traditionnelle, si bien qu'il subsiste un risque que l'implantation finalement réalisée ne soit pas pleinement satisfaisante, pour les raisons développées plus haut.

On retrouve des considérations similaires pour les opérations de resurfaçage glénoïdien de l'omoplate.

EP-A-2 135 576 propose, quant à lui, un dispositif et un procédé permettant d'optimiser le positionnement d'un implant glénoïdien. L'enseignement technique de ce document repose sur une modélisation d'une surface glénoïdienne théorique, étant remarqué que, pour aboutir à une telle modélisation, ce document propose de disposer, par des moyens de cartographie ad hoc, de données de cartographie relatives à une surface glénoidienne de l'omoplate d'un patient, ces données de cartographie étant définies dans un système de repérage spatial de l'omoplate.

Le but de la présente invention est d'optimiser soit la configuration d'implantation d'un composant glénoïdien, soit la configuration d'un outil de resurfaçage glénoïdien, en tenant davantage compte de l'action, sur l'articulation de l'épaule prothésée ou resurfacée d'un patient, des forces d'origine musculaire liées à une activité articulaire régulière de ce patient.

A cet effet, l'invention a pour objet un dispositif de détermination d'une configuration dans laquelle soit un composant glénoïdien d'une prothèse d'épaule est à implanter sur l'omoplate d'un patient, soit un outil de resurfaçage glénoïdien est à appliquer sur l'omoplate d'un patient, ce dispositif étant tel que défini à la revendication 1.

L'invention a également pour objet un procédé de détermination d'une configuration dans laquelle soit un composant glénoïdien d'une prothèse d'épaule est à implanter sur l'omoplate d'un patient, soit un outil de resurfaçage glénoïdien est à appliquer sur l'omoplate d'un patient, ce procédé étant tel que défini à la revendication 10.

Une des idées à la base de l'invention est de chercher à positionner un composant glénoïdien pré-existant non pas lorsque l'articulation de l'épaule du patient à opérer est considérée au repos, mais lorsque cette articulation est considérée soumise à des forces d'origine musculaire liées à une activité régulière de ce patient, typiquement une activité articulaire que ce patient répète plusieurs fois par jour. Pour « remonter » à une caractérisation vectorielle d'une résultante de ces forces, l'invention exploite des données relatives à l'usure constatée de la surface glénoïdienne du patient. Et grâce aux caractéristiques vectorielles de la résultante forces précitée, on peut déterminer une configuration d'implantation du composant prothétique glénoïdien, en considérant l'influence positionnelle de cette résultante de forces sur la coopération articulaire entre l'omoplate et l'humérus du patient. De cette façon, après implantation chirurgicale du composant glénoïdien conformément à la position d'implantation déterminée grâce à l'invention, les efforts liés à l'activité régulière du patient seront appliqués de manière équilibrée sur l'articulation entre l'omoplate prothésée et l'humérus, si bien que le patient pourra reprendre cette activité régulière en limitant le risque que cette dernière conduise rapidement à une instabilité de la prothèse implantée.

L'invention peut être appliquée à un composant glénoïdien d'une prothèse d'épaule totale, la tête de l'humérus du patient étant alors à prothéser par un composant huméral de la prothèse d'épaule. L'invention peut également être appliquée à un composant prothétique glénoïdien destiné à s'articuler directement sur la tête articulaire naturelle de l'humérus du patient. Par ailleurs, l'invention s'applique aussi bien aux composants glénoïdiens dont la face articulaire est concave, qu'aux composants à face articulaire convexe.

De plus, comme spécifié plus haut dans la définition de l'invention, cette dernière peut être mise en œuvre dans le cadre du resurfaçage glénoïdien de l'omoplate d'un patient, typiquement par fraisage. L'invention s'applique alors au positionnement de l'outil de fraisage sur l'omoplate.

On remarquera que toutes les étapes du procédé de détermination défini plus haut peuvent être mises en œuvre de manière pré-opératoire, notamment à l'aide de moyens de calcul et de simulation ad hoc. Le résultat de ce procédé est, quant à lui, exploitable ultérieurement, lors d'une intervention chirurgicale subséquente visant effectivement à implanter un composant glénoïdien ou à appliquer un outil de resurfaçage glénoïdien.

D'autres caractéristiques avantageuses du dispositif de détermination conforme à l'invention, prises isolément ou selon toutes les combinaisons techniquement possibles, sont spécifiées aux revendications dépendantes.

Par ailleurs, le procédé de détermination conforme à l'invention présente avantageusement des aspects additionnels qui correspondent respectivement à la mise en œuvre des caractéristiques additionnelles avantageuses listées ci-dessus pour le dispositif de détermination.

En lien avec l'invention, il est proposé une méthode chirurgicale d'implantation chez un patient d'un composant glénoïdien d'une prothèse d'épaule, dans laquelle :
- on détermine une position préférentielle d'implantation du composant glénoïdien conformément au procédé de détermination tel que défini plus haut,
- on repère dans l'espace l'omoplate du patient, avec un lien univoque avec le système de repérage défini dans le procédé de détermination, et
- on met en place le composant glénoïdien sur l'omoplate du patient selon la position d'implantation préférentielle.

Avantageusement, au moins une partie des données de cartographie sont acquises par palpation de l'omoplate du patient.

Egalement en lien avec l'invention, il est proposé une méthode chirurgicale de resurfaçage glénoïdien de l'omoplate d'un patient, dans laquelle :
- on détermine un positionnement préférentiel d'application d'un outil de resurfaçage conformément au procédé de détermination tel que défini plus haut,
- on repère dans l'espace l'omoplate du patient, avec un lien univoque avec le système de repérage défini dans le procédé de détermination, et
- on rectifie la surface glénoïdienne de l'omoplate en appliquant sur cette dernière l'outil de resurfaçage selon le positionnement d'application préférentiel.

Avantageusement, au moins une partie des données de cartographie sont acquises par palpation de l'omoplate du patient.

L'invention sera mieux comprise à la lecture de la description qui va suivre, donnée uniquement à titre d'exemple et faite en se référant aux dessins sur lesquels :
- la figure 1 est une vue schématique en perspective et en éclaté de l'épaule d'un patient, associée à des composants prothétiques d'une prothèse d'épaule ;
- la figure 2 est une coupe schématique de l'épaule, non prothésée, de la figure 1 ;
- les figures 3 et 4 sont des vues analogues à la figure 2, montrant en élévation deux configurations différentes d'implantation de la prothèse d'épaule de la figure 1 ; et
- la figure 5 est une vue schématique d'un système d'implantation chez le patient du composant glénoïdien de la prothèse d'épaule.

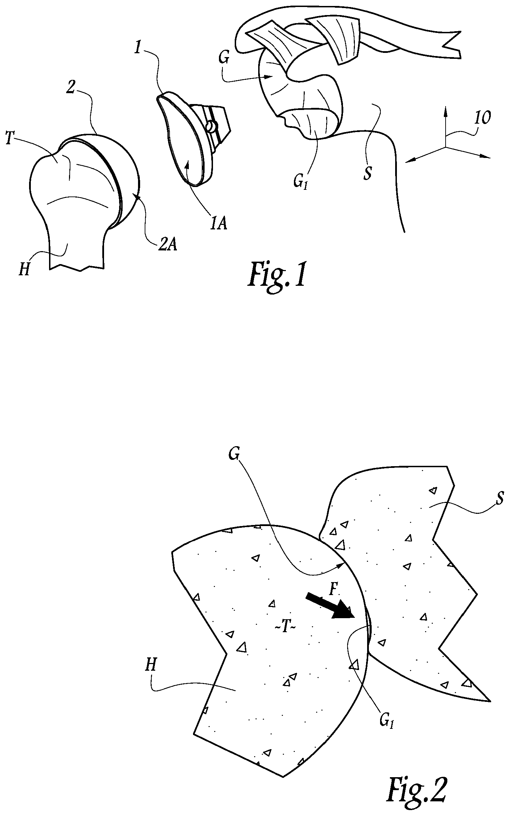

Sur les figures 1 et 2 est montré, en partie, une épaule d'un patient, comprenant une omoplate S et un humérus H. L'omoplate S délimite, sur son côté latéral tourné vers l'humérus H, une surface glénoïdienne G. A l'état naturel de l'épaule, la tête T de l'humérus H s'appuie de manière articulée contre la surface glénoïdienne G. De manière non représentée sur les figures, la coopération articulaire entre l'omoplate S et l'humérus H est commandée par des muscles s'étendant entre l'omoplate et l'humérus, en particulier le muscle deltoïde et les muscles de la coiffe des rotateurs. Par la suite, on utilise l'expression « environnement musculaire » pour désigner les muscles précités.

Sur les figures 1, 3 et 4 est représentée, de manière très schématique, une prothèse d'épaule connue en soi, comprenant un composant glénoïdien 1 et un composant huméral 2. Dans l'exemple non limitatif considéré ici, ces composants glénoïdien 1 et huméral 2 présentent des formes globales respectives de portion de coupelle, en délimitant des faces articulaires respectives 1A et 2A géométriquement complémentaires l'une de l'autre de manière que ces composants s'articulent l'un sur l'autre.

On va décrire ci-après comment déterminer une configuration d'implantation du composant glénoïdien 1 sur l'omoplate S, en tenant compte de l'environnement musculaire de l'épaule.

Pour ce faire, on considère qu'on dispose initialement de données relatives à la géométrie anatomique de l'omoplate S. Ces données sont prévues pour dresser une cartographie tri-dimensionnelle de la surface glénoïdienne G de cette omoplate, après avoir défini un système 10 de repérage dans l'espace de l'omoplate S. Ce système de repérage spatial 10 est par exemple établi à l'aide de points de repère naturels remarquables de cette omoplate.

En pratique, les données de cartographie relatives à la géométrie naturelle de la surface glénoïdienne G sont avantageusement extraites depuis des images pré-opératoires de l'omoplate S, en particulier des images de scanner. La figure 2 correspond, en quelque sorte, à une telle image pré-opératoire.

A partir des données de cartographie précitées, on caractérise l'usure de la surface glénoïdienne G. Ainsi, dans l'exemple considéré sur les figures 1 et 2, la surface glénoïdienne G présente, dans sa partie inférieure et suivant toute sa dimension antéro-postérieure, une zone usée G₁, vue en coupe sur la figure 2, étant remarqué que les usures glénoïdiennes sont plutôt constatées le plus souvent dans la partie supérieure de la surface glénoïdienne.

En pratique, la zone usée G₁ est caractérisée par ses caractéristiques géométriques, appréciées par rapport au reste de la surface glénoïdienne G. Ainsi, à titre non limitatif, la zone usée G₁ est caractérisée par les dimensions de son contour périphérique dans les trois directions du système de repérage 10, par ses gradients de profondeur dans le système de repérage 10, etc. En fait, de manière plus générale, ces caractéristiques géométriques d'usure sont choisies pour permettre, comme expliqué en détail plus loin, de « remonter » aux causes mécaniques à l'origine de l'usure de la surface glénoïdienne G.

Pour déterminer les caractéristiques géométriques d'usure de la surface glénoïdienne G, plusieurs possibilités sont envisageables. L'une d'elles consistent à comparer les données de cartographie relatives à la surface glénoïdienne G, obtenues précédemment, à des données relatives à l'anatomie d'une surface glénoïdienne de référence, de telles données de comparaison étant fournies par une base de données pré-existante.

Une autre possibilité consiste, sur la base des données de cartographie relatives à une partie seulement de la surface glénoïdienne G, considérée comme non usée, à modéliser une surface glénoïdienne théorique, par le biais d'algorithmes de reconnaissance de formes et de données génétiques et morphométriques préétablies, puis de comparer les données de cartographie restantes, en particulier celles relatives à la zone usée G₁, à cette surface glénoïdienne théorique.

L'intérêt de disposer des caractéristiques géométriques d'usure de la surface glénoïdienne G est, lors d'une étape subséquente, d'utiliser ces caractéristiques pour estimer les forces responsables de la formation de la zone usée G₁. En effet, l'apparition et l'évolution de la zone usée G₁ au sein de la surface glénoïdienne G sont la conséquence de l'action régulière et répétitive d'une certaine configuration de l'environnement musculaire de l'épaule du patient : en raison d'une activité articulaire régulière du patient, c'est-à-dire de gestes qu'il répète de manière fréquente dans le cadre de sa vie quotidienne, l'environnement musculaire de l'épaule applique à l'omoplate S et à l'humérus H des contraintes répétées qui, à la longue, entraînent l'apparition et le développement de l'usure de la surface glénoïdienne G au niveau de la zone G₁. Cette action de l'environnement musculaire peut être représentée par une résultante de forces, notée F sur la figure 2, dont les caractéristiques vectorielles sont déterminées à partir des caractéristiques géométriques d'usure de la surface glénoïdienne G.

En pratique, pour « remonter » aux caractéristiques vectorielles de la résultante de forces F à partir des caractéristiques géométriques d'usure de la surface glénoïdienne G, on utilise avantageusement un modèle musculo-squelettique d'épaule pré-existant. Un tel modèle bio-mécanique permet de simuler les mouvements articulaires de l'épaule, en quantifiant les forces dans l'articulation entre l'omoplate et l'humérus de l'épaule, ainsi que dans l'environnement musculaire de l'épaule. De cette façon, un tel modèle musculo-squelettique d'épaule peut être utilisé pour construire une base de données d'usure : pour ce faire, on simule plusieurs usures glénoïdiennes au sein de ce modèle, respectivement sous l'action de différentes forces prédéterminées correspondantes. Par comparaison des caractéristiques géométriques d'usure de la surface glénoïdienne G à la base de données d'usure ainsi pré-établie, on déduit, le cas échéant par approximation, les caractéristiques vectorielles de la résultante de forces F.

Enfin, en utilisant les caractéristiques vectorielles de la résultante de forces F, on détermine une position optimale d'implantation du composant glénoïdien 1 sur l'omoplate S de telle sorte que, en service ultérieur, le composant glénoïdien s'oppose à la résultante de forces F. Autrement dit, on tient ainsi compte de l'action de cette résultante de forces F sur la coopération articulaire à venir entre le composant glénoïdien 1 et le composant huméral 2, selon la configuration relative d'implantation de ces composants au sein de l'épaule du patient. Pour ce faire, selon une mise en œuvre préférée, on utilise de nouveau le modèle musculo-squelettique d'épaule évoqué plus haut, pour simuler l'articulation entre l'omoplate S et l'humérus H, soumise à des efforts musculaires correspondant à la résultante F et calculer alors, dans le système de repérage 10, les caractéristiques géométriques d'une position d'implantation du composant glénoïdien 1 de manière que la mobilité relative entre ce dernier et le composant huméral 2 soit équilibrée sous l'effet de la résultante des forces F. En pratique, cet équilibrage est avantageusement déterminé de sorte que, au cours des mouvements de l'épaule prothésée produisant des efforts de résultante F, la zone de contact articulaire entre l'omoplate S et l'humérus H est sensiblement centrée par rapport au contour périphérique du composant glénoïdien, et non décalée vers une portion périphérique de ce dernier.

Ainsi, les caractéristiques géométriques précitées, relatives à la position d'implantation du composant glénoïdien 1, permettent de quantifier tous les paramètres de positionnement vis-à-vis de l'omoplate S dans le système de repérage 10, à savoir sa hauteur dans les trois directions de ce système de repérage, ainsi que son inclinaison selon ces trois directions.

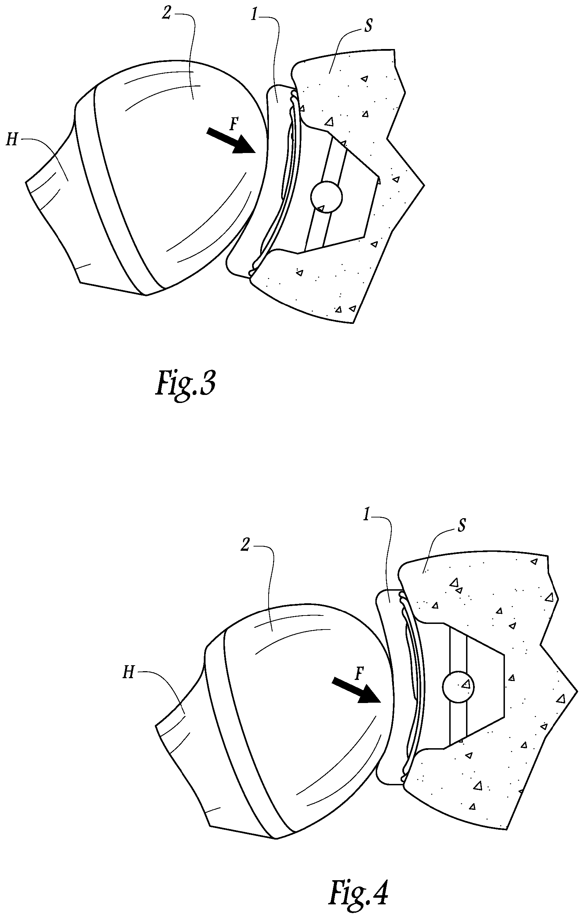

La position d'implantation du composant glénoïdien 1 ainsi déterminée est montrée sur la figure 3 qui illustre l'équilibrage entre composant glénoïdien 1 et le composant huméral 2 alors que l'omoplate S et l'humérus H de l'épaule du patient à opérer subissent l'effet de la résultante de forces F. A l'inverse, la figure 4 illustre une autre configuration d'implantation selon laquelle le composant glénoïdien 1 est placé sans tenir compte de la résultante de forces F : dans ce cas, contrairement à la figure 3, on constate que la position du composant 1 n'équilibre pas de façon satisfaisante l'action de la résultante F sur la mobilité de l'épaule prothésée, si bien que, dès que le patient reprendra ses activités de tous les jours et à chaque fois qu'il répètera les gestes ayant conduit à l'apparition de la zone usée G₁ de sa surface glénoïdienne G, la prothèse d'épaule sera sollicitée selon une configuration non équilibrée entre ses composants 1 et 2, d'où, à la longue, un risque significatif d'instabilité de la prothèse.

Ainsi, à l'issue du procédé qui vient d'être décrit, on a déterminé une position préférentielle d'implantation du composant glénoïdien 1 sur l'omoplate S, position préférentielle qui tient compte de l'action de la résultante de forces F sur l'articulation à prothéser entre l'omoplate et l'humérus H du patient. On souligne ici que les différentes étapes de ce procédé peuvent être mises en œuvre en dehors d'une intervention chirurgicale, proprement dite, visant à implanter le composant glénoïdien 1, dans le sens où ces étapes sont mises en œuvre sans avoir à accéder réellement à l'omoplate S et à l'humérus H du patient, notamment via des incisions des parties molles entourant ces os.

Par ailleurs, en pratique, la mise en œuvre des étapes décrites jusqu'ici sont avantageusement assistées par des moyens informatiques, en particulier pour réaliser les calculs de positionnement relatif et les calculs de simulation évoqués jusqu'ici.

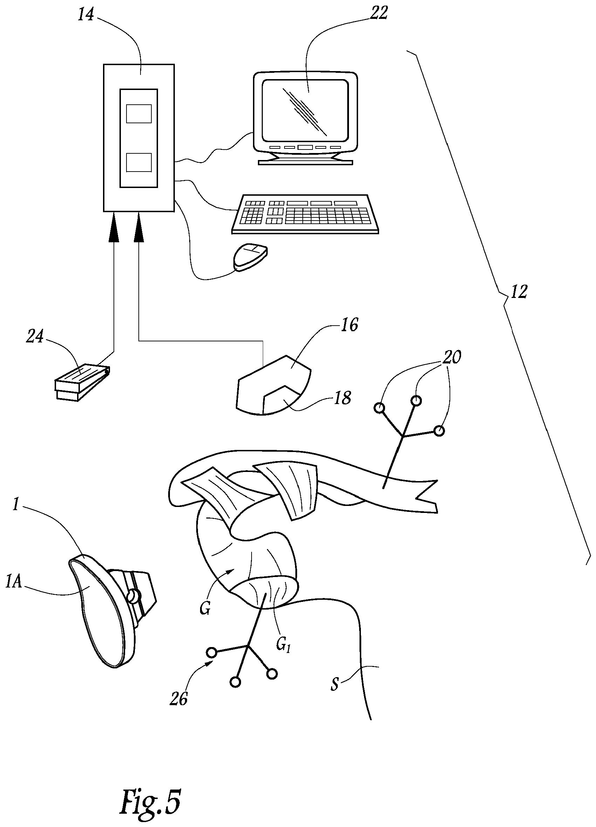

Ultérieurement, un chirurgien va utiliser les données, obtenues par le procédé décrit jusqu'ici, relatives à la position préférentielle d'implantation du composant glénoïdien 1. A cette fin, le chirurgien utilise notamment un ensemble chirurgical 12 montré sur la figure 5 : cet ensemble 12 comprend un ordinateur 14 associé à une unité d'émission et de réception d'un rayonnement infrarouge, cette unité comportant un capteur 16 relié à l'ordinateur et une source d'alimentation infrarouge 18 couvrant le champ opératoire dans lequel est représentée l'omoplate S du patient. Pour permettre à l'ordinateur 14 de repérer dans l'espace l'omoplate S, l'ensemble 12 comporte un groupe de marqueurs 20 qui renvoient, de façon passive, le rayonnement infrarouge en direction du capteur 16. Ce groupe de marqueurs 20 forme un système de marquage tri-dimensionnel permettant aux capteurs 16 de suivre dans l'espace la position de l'omoplate. L'utilisation de tels marqueurs est bien connue dans le domaine de l'orthopédie assistée par ordinateur, par exemple dans le document EP-A-1 249 213, de sorte que ces marqueurs ne seront pas décrits ici plus avant.

Avantageusement, l'ordinateur 14 est également associé à un écran vidéo 22 à même d'afficher des informations utiles au chirurgien, notamment les informations relatives au repérage de l'omoplate S et d'autres données décrites plus loin, de préférence sous forme de représentations graphiques. L'ensemble 12 comprend également des moyens de commande 24, par exemple sous forme d'une pédale actionnable par le pied du chirurgien.

En vue d'implanter le composant glénoïdien 1 dans la position d'implantation préférentielle précitée, on établit, grâce à l'ordinateur 14, un lien univoque entre le repérage spatial à l'aide du groupe de marqueurs 20 et le système de repérage 10 utilisé pour la mise en œuvre du procédé de détermination de cette position d'implantation préférentielle. Pour ce faire, le chirurgien utilise, à titre d'exemple, un palpeur 26 qui est repéré par le capteur 16, moyennant un étalonnage préalable : après incision des parties molles de l'épaule du patient, le chirurgien amène ce palpeur 26 au moins sur les lieux remarquables de l'omoplate S ayant servi à définir le système de repérage 10 et, par actionnement de la pédale de commande 24, le chirurgien fait enregistrer à l'ordinateur 14 la position du palpeur 26. Puis, à partir de ces données, l'ordinateur 14 est à même de calculer le lien mathématique entre le système de repérage 10 et le repérage spatial des capteurs 16.

Le chirurgien met ensuite en place le composant glénoïdien 1 sur l'omoplate S selon la position d'implantation préférentielle. En pratique, les gestes correspondants du chirurgien sont avantageusement guidés par des moyens de navigation pilotés par l'ordinateur 14. Cet aspect de guidage chirurgical est bien connu dans le domaine orthopédique et ne sera donc pas décrit ici plus avant.

A titre optionnel, après que le chirurgien a incisé les parties molles de l'épaule du patient mais avant qu'il commence à mettre en place le composant glénoïdien 1, le chirurgien peut mettre à profit son accès à l'omoplate S pour recueillir des données de cartographie relatives à la surface glénoïdienne G. Ces données de cartographie pourront ainsi compléter, voire constituer en totalité les données de cartographie utilisées pour la mise en œuvre du procédé de détermination de la position d'implantation préférentielle du composant glénoïdien 1 : autrement dit, dans ce cas, la mise en œuvre de ce procédé de détermination est réalisée de manière per-opératoire, contrairement au cas de figure évoqué plus haut où la mise en œuvre de ce procédé a été décrite comme étant de manière pré-opératoire.

A titre d'exemple, les données de cartographie relatives à l'omoplate S sont ainsi obtenues en per-opératoire à l'aide du palpeur 26 amené sur la surface glénoïdienne G.

Divers aménagements et variantes au procédé de détermination et au dispositif permettant sa mise en œuvre, ainsi qu'à la méthode chirurgicale d'implantation et à l'ensemble permettant la mise en œuvre de cette méthode, sont par ailleurs envisageables. A titre d'exemples :
- les moyens de repérage de l'omoplate S et/ou du palpeur 26 ne sont pas limités à des marqueurs réfléchissant l'infrarouge ; les marqueurs sensibles aux ultrasons ou à des champs électromagnétiques sont notamment utilisables ;
- plutôt que la position d'implantation du composant huméral 2 sur l'humérus H soit prédéterminée, cette implantation peut être réglée concomitamment à la détermination d'une configuration d'implantation du composant glénoïdien 1 ;
- comme évoqué dans la partie introductive de ce document, l'invention peut être mise en œuvre pour déterminer une configuration d'implantation préférentielle d'un composant glénoïdien s'articulant directement sur la tête naturelle de l'humérus H, sans nécessiter l'implantation d'un composant huméral tel que le composant 2 ; et/ou
- l'invention peut également être mise en œuvre dans le cadre du resurfaçage glénoïdien de l'omoplate S, avec ou sans mise en place subséquente d'un implant de resurfaçage ; dans ce cas, plutôt que de déterminer une position d'implantation du composant glénoïdien 1, comme décrit plus haut, l'invention est appliquée pour déterminer un positionnement d'application d'un outil de resurfaçage sur l'omoplate, afin de rectifier sa surface glénoïdienne G pour que cette dernière puisse ensuite s'articuler au mieux avec la tête de l'humérus H, c'est-à-dire en tenant compte de l'action de la résultante de forces F sur cette articulation ; les considérations détaillées jusqu'ici quant à la détermination d'une configuration d'implantation du composant glénoïdien 1 s'appliquent mutatis mutandis à la détermination d'une configuration de positionnement de l'outil de resurfaçage sur l'omoplate.

## Revendications

1. Dispositif de détermination d'une configuration dans laquelle soit un composant glénoïdien (1) d'une prothèse d'épaule est à implanter sur l'omoplate (S) d'un patient, soit un outil de resurfaçage glénoïdien est à appliquer sur l'omoplate d'un patient, ce dispositif comportant :
- des moyens de cartographie pour disposer de données de cartographie relatives à la surface glénoïdienne (G) de l'omoplate (S), ces données de cartographie étant définies dans un système de repérage spatial (10) de l'omoplate,
- des premiers moyens de détermination informatiques, prévus pour, à partir de données de cartographie fournies par les moyens de cartographie, déterminer des caractéristiques géométriques d'usure de la surface glénoïdienne (G),
- des deuxièmes moyens de détermination informatiques, prévus pour, à partir de caractéristiques géométriques d'usure fournies par les premiers moyens de détermination, déterminer les caractéristiques vectorielles d'une résultante (F) de forces responsables de l'usure de la surface glénoïdienne (G), et
- des troisièmes moyens de détermination informatiques, prévus pour, à partir des caractéristiques vectorielles d'une résultante de forces (F) fournies par les deuxièmes moyens de détermination, déterminer soit une position d'implantation du composant glénoïdien (1) sur l'omoplate (S), soit un positionnement d'application de l'outil de resurfaçage sur l'omoplate, en tenant compte de l'action de ladite résultante de forces sur la coopération articulaire entre l'omoplate, respectivement prothésée ou resurfacée, et l'humérus (H) du patient.

2. Dispositif suivant la revendication 1, **caractérisé en ce que** les moyens de cartographie comprennent des images pré-opératoires, telles que des images de scanner.

3. Dispositif suivant l'une des revendications 1 ou 2, **caractérisé en ce que** les premiers moyens de détermination sont adaptés pour comparer les données de cartographie à des données relatives à une anatomie de référence pour une surface glénoïdienne, fournies par une base de données pré-existante.

4. Dispositif suivant l'une des revendications 1 ou 2, **caractérisé en ce qu'**il comporte en outre des moyens pour modéliser une surface glénoïdienne théorique à partir d'une partie seulement des données de cartographie, cette partie des données de cartographie correspondant sélectivement à une partie seulement de la surface glénoïdienne (G) de l'omoplate (S), et **en ce que** les premiers moyens de détermination sont adaptés pour comparer le reste des données de cartographie à la surface glénoïdienne théorique fournie par les moyens de modélisation.

5. Dispositif suivant l'une quelconque des revendications précédentes, **caractérisé en ce que** les deuxièmes moyens de détermination sont adaptés pour comparer les caractéristiques géométriques d'usure à des données d'usure pré-établies à l'aide d'un modèle musculo-squelettique d'épaule pré-existant.

6. Dispositif suivant la revendication 5, **caractérisé en ce que** lesdites données d'usure pré-établies sont obtenues en simulant des usures glénoïdiennes du modèle musculo-squelettique d'épaule sous les actions respectives de forces prédéterminées correspondantes.

7. Dispositif suivant l'une quelconque des revendications précédentes, **caractérisé en ce que** les troisièmes moyens de détermination sont adaptés pour déterminer soit la position d'implantation du composant glénoïdien (1), soit le positionnement d'application de l'outil de resurfaçage de telle sorte que la coopération articulaire entre l'omoplate (S), respectivement prothésée ou resurfacée, et l'humérus (H) soit équilibrée sous l'effet d'une force conforme aux caractéristiques vectorielles de ladite résultante de forces (F).

8. Dispositif suivant la revendication 7, **caractérisé en ce que** les troisièmes moyens de détermination incluent des moyens de calcul pour, à partir d'un modèle musculo-squelettique d'épaule pré-existant, simuler la coopération articulaire entre l'omoplate (S) et l'humérus (H), soumise à une force conforme aux caractéristiques vectorielles de ladite résultante de forces (F).

9. Dispositif suivant l'une des revendications 7 ou 8, **caractérisé en ce que** les troisième moyens de détermination sont adaptés pour calculer les caractéristiques géométriques, dans le système de repérage spatial (10), soit de la position d'implantation du composant glénoïdien (1), soit du positionnement d'application de l'outil de resurfaçage de telle sorte que, lorsque l'épaule du patient se trouve sollicitée de façon à appliquer à l'articulation entre l'omoplate (S) et l'humérus (H) une force conforme aux caractéristiques vectorielles de ladite résultante de forces (F), la zone de contact articulaire entre l'omoplate, respectivement prothésée ou resurfacée, et l'humérus est sensiblement centrée par rapport au contour périphérique du composant glénoïdien ou du resurfaçage.

10. Procédé de détermination d'une configuration dans laquelle soit un composant glénoïdien (1) d'une prothèse d'épaule est à implanter sur l'omoplate (S) d'un patient, soit un outil de resurfaçage glénoïdien est à appliquer sur l'omoplate d'un patient, procédé dans lequel :
- on définit un système de repérage spatial (10) de l'omoplate (S),
- on dispose de données de cartographie relatives à la surface glénoïdienne (G) de cette omoplate (S), ces données de cartographie étant définies dans le système de repérage (10),
- à partir de ces données de cartographie, on détermine, par des moyens informatiques, des caractéristiques géométriques d'usure de la surface glénoïdienne (G),
- à partir de ces caractéristiques géométriques d'usure, on détermine, par des moyens informatiques, les caractéristiques vectorielles d'une résultante (F) de forces responsables de l'usure de la surface glénoïdienne (G), et
- à partir de ces caractéristiques vectorielles de la résultante de forces (F), on détermine, par des moyens informatiques, soit une position d'implantation du composant glénoïdien (1) sur l'omoplate (S), soit un positionnement d'application de l'outil de resurfaçage sur l'omoplate, en tenant compte de l'action de cette résultante de forces sur la coopération articulaire entre l'omoplate, respectivement prothésée ou resurfacée, et l'humérus (H) du patient.

11. Procédé selon la revendication 10, dans lequel les données relatives à soit ladite position d'implantation du composant glénoïdien (1) sur l'omoplate (S), soit ledit positionnement d'application de l'outil de resurfaçage sur l'omoplate, sont fournies à un ordinateur (14) associé à des moyens (16, 18, 20) de repérage spatial de l'omoplate (S).

## Patentansprüche

1. Vorrichtung zur Bestimmung einer Konfiguration, in der entweder eine glenoidale Komponente (1) einer Schulterprothese an dem Schulterblatt (S) eines Patienten implantiert werden soll oder ein Werkzeug zur glenoidalen Oberflächenbearbeitung an dem Schulterblatt eines Patienten angebracht werden soll, wobei diese Vorrichtung umfasst:
- Kartographiemittel zum Aufstellen von Kartographiedaten bezüglich der glenoidalen Oberfläche (G) des Schulterblatts (S), wobei diese Kartographiedaten in einem räumlichen Ortungssystem (10) des Schulterblatts definiert sind,
- erste rechnergestützte Bestimmungsmittel, die vorgesehen sind, aus den von den Kartographiemitteln gelieferten Kartographiedaten geometrische Abnutzungsmerkmale der glenoidalen Oberfläche (G) zu bestimmen,
- zweite rechnergestützte Bestimmungsmittel, die vorgesehen sind, aus den von den ersten rechnergestützten Bestimmungsmitteln gelieferten geometrischen Abnutzungsmerkmalen die vektoriellen Charakteristika einer Resultierenden (F) von Kräften, die für die Abnutzung der glenoidalen Oberfläche (G) verantwortlich sind, zu bestimmen, und
- dritte rechnergestützte Bestimmungsmittel, die vorgesehen sind, aus den von den zweiten rechnergestützten Bestimmungsmitteln gelieferten vektoriellen Charakteristika einer Resultierenden von Kräften (F) entweder eine Position zur Implantation der glenoidalen Komponente (1) an dem Schulterblatt (S) oder eine Positionierung für die Anwendung des Werkzeugs zur Oberflächenbearbeitung an dem Schulterblatt zu bestimmen, wobei die Wirkung der Resultierenden von Kräften auf die Gelenkzusammenarbeit zwischen dem jeweilig mit einer Prothese versehenen oder oberflächenbearbeiteten Schulterblatt und dem Oberarmknochen (H) des Patienten berücksichtigt wird.

2. Vorrichtung nach Anspruch 1, **dadurch gekennzeichnet, dass** die Kartographiemittel präoperative Bilder, wie Scanner-Bilder, umfassen.

3. Vorrichtung nach einem der Ansprüche 1 oder 2, **dadurch gekennzeichnet, dass** die ersten Bestimmungsmittel angepasst sind, die Kartographiedaten mit Daten zu vergleichen, die sich auf eine Referenzanatomie für eine Glenoidoberfläche beziehen, die von einer bereits existierenden Datenbank geliefert werden.

4. Vorrichtung nach einem der Ansprüche 1 oder 2, **dadurch gekennzeichnet, dass** sie ferner Mittel zum Modellieren einer theoretischen Glenoidoberfläche aus nur einem Teil der Kartographiedaten umfasst, wobei dieser Teil der Kartographiedaten selektiv nur einem Teil der Glenoidoberfläche (G) des Schulterblatts (S) entspricht, und dass die ersten Bestimmungsmittel angepasst sind, den Rest der Kartographiedaten mit der theoretischen Glenoidoberfläche zu vergleichen, die von den Mitteln zum Modellieren geliefert wird.

5. Vorrichtung nach einem der vorhergehenden Ansprüche, **dadurch gekennzeichnet, dass** die zweiten Bestimmungsmittel angepasst sind, die geometrischen Abnutzungsmerkmale mit zuvor ermittelten Abnutzungsdaten unter Verwendung eines bereits vorhandenen Muskel-Skelett-Schultermodells zu vergleichen.

6. Vorrichtung nach Anspruch 5, **dadurch gekennzeichnet, dass** die zuvor ermittelten Abnutzungsdaten durch Simulation der glenoidalen Abnutzung des Muskel-Skelett-Schultermodells unter den jeweiligen Einwirkungen entsprechender vorbestimmter Kräfte erhalten werden.

7. Vorrichtung nach einem der vorhergehenden Ansprüche, **dadurch gekennzeichnet, dass** die dritten Bestimmungsmittel angepasst sind, entweder die Position der Implantation der glenoidalen Komponente (1) oder die Positionierung für die Anwendung des Werkzeugs zur Oberflächenbearbeitung so zu bestimmen, dass die Gelenkzusammenarbeit zwischen dem jeweilig mit einer Prothese versehenen oder oberflächenbearbeiteten Schulterblatt (S) und dem Oberarmknochen (H) unter der Einwirkung einer Kraft, die den vektoriellen Charakteristika der genannten Resultierenden der Kräfte (F) entspricht, ausgeglichen ist.

8. Vorrichtung nach Anspruch 7, **dadurch gekennzeichnet, dass** die dritten Bestimmungsmittel Berechnungsmittel umfassen, um auf der Grundlage eines bereits vorhandenen Muskel-Skelett-Schultermodells die Gelenkzusammenarbeit zwischen dem Schulterblatt (S) und dem Oberarmknochen (H) zu simulieren, die einer Kraft gemäß den vektoriellen Charakteristika der genannten Resultierenden der Kräfte (F) unterworfen ist.

9. Vorrichtung nach einem der Ansprüche 7 oder 8, **dadurch gekennzeichnet, dass** die dritten Bestimmungsmittel angepasst sind, die geometrischen Merkmale entweder der Position der Implantation der glenoidalen Komponente (1) oder der Positionierung für die Anwendung des Werkzeugs zur Oberflächenbearbeitung in dem räumlichen Ortungssystem (10) so zu berechnen, dass, wenn die Schulter des Patienten so belastet wird, dass auf das Gelenk zwischen dem Schulterblatt (S) und dem Oberarmknochen (H) eine Kraft ausgeübt wird, die den vektoriellen Charakteristika der resultierenden Kraft (F) entspricht, die Zone des Gelenkkontakts zwischen dem jeweilig mit einer Prothese versehenen oder oberflächenbearbeiteten Schulterblatt und dem Oberarmknochen in Bezug auf die Umfangskontur der glenoidalen Komponente oder der Oberflächenbearbeitung im Wesentlichen zentriert ist.

10. Verfahren zum Bestimmen einer Konfiguration, in der entweder eine glenoidale Komponente (1) einer Schulterprothese an dem Schulterblatt (S) eines Patienten implantiert werden soll oder ein Werkzeug zur glenoidalen Oberflächenbearbeitung an dem Schulterblatt eines Patienten angebracht werden soll, wobei bei dem Verfahren:
- ein räumliches Ortungssystem (10) des Schulterblatts (S) definiert wird,
- Kartographiedaten bezüglich der glenoidalen Oberfläche (G) dieses Schulterblatts (S) aufgestellt werden, wobei diese Kartographiedaten in dem räumlichen Ortungssystem (10) des Schulterblatts definiert werden,
- aus diesen Kartographiedaten mit Hilfe von rechnergestützten Mitteln geometrische Abnutzungsmerkmale der glenoidalen Oberfläche (G) ermittelt werden,
- aus diesen geometrischen Abnutzungsmerkmalen die vektoriellen Charakteristika einer Resultierenden (F) von Kräften mit den rechnergestützten Mitteln bestimmt werden, die für die Abnutzung der glenoidalen Oberfläche (G) verantwortlich sind, und
- aus den vektoriellen Charakteristika der Resultierenden von Kräften (F) mit den rechnergestützten Mitteln entweder eine Position zur Implantation der glenoidalen Komponente (1) an dem Schulterblatt (S) oder eine Positionierung der Anwendung des Werkzeugs zur Oberflächenbearbeitung an dem Schulterblatt bestimmt werden, wobei die Wirkung dieser Resultierenden von Kräften auf die Gelenkzusammenarbeit zwischen dem jeweilig mit einer Prothese versehenen oder oberflächenbearbeiteten Schulterblatt und dem Oberarmknochen (H) des Patienten berücksichtigt wird.

11. Verfahren nach Anspruch 10, bei dem die Daten, die sich entweder auf die Position zur Implantation der glenoidalen Komponente (1) an dem Schulterblatt (S) oder die Positionierung der Anwendung des Werkzeugs zur Oberflächenbearbeitung an dem Schulterblatt beziehen, einem Rechner (14) zugeführt werden, der Mitteln (16, 18, 20) zur räumlichen Ortung des Schulterblatts (S) zugeordnet ist.

## Claims

1. Device for determining a configuration in which either a glenoid component (1) of a shoulder prosthesis is to be implanted on the scapula (S) of a patient, or a glenoid resurfacing tool is to be applied to the scapula of a patient, this device comprising:
- mapping means in order to have mapping data relating to the glenoid surface (G) of the scapula (S), these mapping data being defined in a spatial coordinate system (10) of the scapula,
- first determination computer means for, from mapping data supplied by the mapping means, determining geometric characteristics of wear of the glenoid surface (G),
- second determination computer means for, from geometric characteristics of wear supplied by the first determination means, determining the vector characteristics of a resultant (F) of forces responsible for the wear of the glenoid surface (G), and
- third determination computer means for, from the vector characteristics of a resultant of forces (F) supplied by the second determination means, determining either a position of implantation of the glenoid component (1) on the scapula (S), or a position of application of the resurfacing tool on the scapula, by taking into account the action of said resultant of forces on the articular cooperation between the scapula, respectively prosthesized or resurfaced, and the humerus (H) of the patient.

2. Device according to Claim 1, **characterized in that** the mapping means comprise preoperational images, such as scanner images.

3. Device according to one of Claims 1 or 2, **characterized in that** the first determination means are designed to compare the mapping data to data relating to a reference anatomy for a glenoid surface, supplied by a pre-existing database.

4. Device according to one of Claims 1 or 2, **characterized in that** it also comprises means for modeling a theoretical glenoid surface from only a portion of the mapping data, this portion of the mapping data selectively corresponding to only a portion of the glenoid surface (G) of the scapula (S), and **in that** the first determination means are designed to compare the rest of the mapping data to the theoretical glenoid surface supplied by the modeling means.

5. Device according to any one of the preceding claims, **characterized in that** the second determination means are designed to compare the geometric characteristics of wear to wear data pre-established using a pre-existing shoulder musculoskeletal model.

6. Device according to Claim 5, **characterized in that** said pre-established wear data are obtained by simulating glenoid wear of the shoulder musculoskeletal model under the respective actions of corresponding predetermined forces.

7. Device according to any one of the preceding claims, **characterized in that** the third determination means are designed to determine either the position of implantation of the glenoid component (1), or the position of application of the resurfacing tool so that the articular cooperation between the scapula (S), respectively prosthesized or resurfaced, and the humerus (H) are balanced under the effect of a force conforming to the vector characteristics of said resultant of forces (F).

8. Device according to Claim 7, **characterized in that** the third determination means include calculation means for, from a pre-existing shoulder musculoskeletal model, simulating the articular cooperation between the scapula (S) and the humerus (H), subjected to a force conforming to the vector characteristics of said resultant of forces (F).

9. Device according to one of Claims 7 or 8, **characterized in that** the third determination means are designed to calculate the geometric characteristics, in the spatial coordinate system (10), either of the position of implantation of the glenoid component (1), or of the position of application of the resurfacing tool so that, when the shoulder of the patient is stressed so as to apply to the articulation between the scapula (S) and the humerus (H) a force conforming to the vector characteristics of said resultant of forces (F), the articular contact region between the scapula, respectively prosthesized or resurfaced, and the humerus is substantially centered relative to the peripheral outline of the glenoid component or of the resurfacing.

10. Method for determining a configuration in which either a glenoid component (1) of a shoulder prosthesis is to be implanted on the scapula (S) of a patient, or a glenoid resurfacing tool is to be applied to the scapula of a patient, a method in which:
- a spatial coordinate system (10) of the scapula (S) is defined,
- mapping data is available relating to the glenoid surface (G) of this scapula (S), these mapping data being defined in the spatial coordinate system (10),
- from these mapping data, geometric characteristics of wear of the glenoid surface (G) are determined by computer means,
- from these geometric characteristics of wear, the vector characteristics of a resultant (F) of forces responsible for the wear of the glenoid surface (G) are determined by computer means, and
- from these vector characteristics of the resultant of forces (F), there is determined by computer means either a position of implantation of the glenoid component (1) on the scapula (S), or a position of application of the resurfacing tool on the scapula, by taking into account the action of this resultant of forces on the articular cooperation between the scapula, respectively prosthesized or resurfaced, and the humerus (H) of the patient.

11. Method according to claim 10, wherein the data relating to either said position of implantation of the glenoidal component (1) on the scapula (S) or said position of application of the resurfacing tool on the scapula are supplied to a computer (14) associated with means (16, 18, 20) for spatial location of the scapula (S).
